# EUROPEAN PATENT APPLICATION

(11) **EP 3 037 008 A1**
(43) Date of publication of application: **29.06.2016**
(21) Application number: 13891999.8
(22) Date of filing: 23.08.2013
(51) Int. Cl.: A24F 47/00

(54) **ATOMIZATION ASSEMBLY AND ELECTRONIC CIGARETTE**

(71) Applicant: KIMREE HI-TECH INC., Road Town, Tortola (VG)
(72) Inventor: LIU, Qiuming, Shenzhen Guangdong 518000 (CN)
(74) Representative: Arpe Fernandez, Manuel de
(86) International application number: PCT/CN2013/082236
(87) International publication number: WO 2015/024269

(57) **Abstract**

An atomization assembly and electronic cigarette are provided. The atomization assembly is used for combining with a battery assembly to form an electronic cigarette. The atomization assembly comprises an outer sleeve (1), and a suction nozzle cover (2) inserted at one end of the outer sleeve (1). A sealing component (3), which is fixedly connected to the suction nozzle cover (2), is provided and protrudes radially from a circumferential outer side wall of the suction nozzle cover (2). The hardness of the sealing component (3) is smaller than that of the suction nozzle cover (2). The suction nozzle cover (2) is integrated with the sealing component (3) into one piece by double-shot molding, so that the suction nozzle cover (2) has a sealing function. A gap between the engagement surfaces of the suction nozzle cover (2) and the outer sleeve (1) is sealed, thereby preventing leakage.

## Description

### TECHNICAL FIELD

The present application relates to the technical field of daily electronic products, and more particularly, relates to an atomization assembly and an electronic cigarette.

### BACKGROUND

Electronic cigarettes are used for smoking cessation and substitute for traditional cigarettes. An electronic cigarette available in the market includes a battery assembly and an atomization assembly. The battery assembly includes a battery of which two ends are provided with a positive pole and a negative pole respectively, and a control board. The control board is further integrated with components including a gas flow sensor, a control circuit and the like. The atomization assembly includes heating wires and tobacco tar. When a smoking action is taking place, the control board controls the battery to supply power to the atomization assembly, thereby driving the heating wires to give out heat to further produce smoke.

Therefore, the battery of the electronic cigarette needs to supply power to the control board and the atomization assembly. In order to make the battery supply power to the control board and atomization assembly, the battery needs to be connected to the control board via wires by means of soldering in order to achieve an electrical connection between the battery and the control board. Besides, corresponding wires need to be soldered between the battery and the atomization assembly, in order to achieve a conduction therebetween.

However, in an existing electronic cigarette, during the assembly of the battery, the wires are soldered manually; therefore, the assembly thereof is inconvenient and time-consuming, which is bad for the automatic production of the electronic cigarette, and the efficiency is low.

Electronic cigarettes are used for smoking cessation and substitute for traditional cigarettes. An electronic cigarette available in the market includes a battery assembly and an atomization assembly. The battery assembly includes a battery and a control module, and the control module is further integrated with components including a gas flow sensor, a control circuit and the like. When smoking via a suction nozzle cover of the atomization assembly, the control module controls the battery to supply power to the atomization assembly, thereby driving the heating wires to give out heat to further produce smoke.

In the prior art, generally, the suction nozzle cover is directly mounted on an outer sleeve. However, various defects are inevitably occurred on surfaces of the suction nozzle cover and the outer sleeve; besides, deviations exist in shapes and dimensions respectively of the suction nozzle cover and the outer sleeve. Therefore, a gap will be inevitably formed at a junction between the suction nozzle cover and the outer sleeve. However, the suction nozzle cover does not have a sealing function, and thus the tobacco tar will leak out via the gap when pressure differences exist on two sides of the junction. In other cases, in the prior art, a movable sealing component is arranged or sleeved on the suction nozzle cover. However, since the sealing ring is movably mounted, frequently, the sealing ring is not arranged in place, thereby causing a leakage of tobacco tar, and thus the user experience is poor.

### BRIEF SUMMARY

The objective of the present application is to provide an electronic cigarette of which the suction nozzle cover has a good sealing effect, aiming at the detects in the prior art that the suction nozzle cover of the electronic cigarette does not have the sealing function.

In accordance with one aspect of the present application, an atomization assembly is constructed, which is configured to be assembled with a battery assembly to form an electronic cigarette, wherein the atomization assembly comprises an outer sleeve and a suction nozzle cover inserted at one end of the outer sleeve; wherein a sealing component, which is fixedly connected to the suction nozzle cover, is protruded radially from a circumferential outer side wall of the suction nozzle cover, and the outer side wall is in contact with the outer sleeve, and a hardness of the sealing component is smaller than that of the suction nozzle cover.

In the atomization assembly of the present application, wherein the outer side wall of the suction nozzle cover is attached to an inner side wall of the outer sleeve; the sealing component extends from the outer side wall of the suction nozzle cover in a direction away from a central axis of the outer sleeve, and is further abutted against the inner side wall of the outer sleeve.

In the atomization assembly of the present application, wherein the sealing component is coaxially sleeved on the circumferential outer side wall of the suction nozzle cover, and the sealing component and the inner side wall of the outer sleeve form interference fit.

In the atomization assembly of the present application, wherein at least one groove adapted to the sealing component is defined on the circumferential outer side wall of the suction nozzle cover;
the sealing component is coaxially sleeved in the groove, and the sealing component and the inner wall of the outer sleeve form interference fit.

In the atomization assembly of the present application, wherein a plurality of grooves are provided; the plurality of grooves are parallel to each other, and are separately arranged on an outer wall of the connection portion; each of the grooves is coaxial with the suction nozzle cover.

In the atomization assembly of the present application, wherein the suction nozzle cover is fixed together with or integrated with the sealing component into one piece by means of double-shot molding or bonding; a cross-section of the sealing component is in shape of an "O", a "V", or a "Y".

In the atomization assembly of the present application, wherein a vent pipe is defined in the outer sleeve; the vent pipe is communicated with air holes defined in the suction nozzle cover to form a smoke channel.

In the atomization assembly of the present application, wherein the suction nozzle cover is in shape of a hollow barrel adapted to the outer sleeve; the air holes are defined in the suction nozzle cover, and the air holes are second air holes.

In the atomization assembly of the present application, wherein the suction nozzle cover includes a cover body opposite to an opening end face of the outer sleeve, and a connection portion embedded in the outer sleeve.

In the atomization assembly of the present application, wherein the connection portion is in shape of a hollow cylinder adapted to the outer sleeve; a second air hole is defined in the connection portion;
the air holes further include a first air hole communicated with the second air hole; the first air hole is defined on an end face of the cover body, and a central axis of the first air hole is aligned with or deviated from a central axis of the outer sleeve;
a flange is formed by extending from an edge of the cover body in a radial direction of the cover body; the flange is abutted against a side edge on an opening end face of the outer sleeve.

In the atomization assembly of the present application, wherein a boss is formed by extending from a central portion on an end face of the cover body that faces to the vent pipe in an axis direction of the cover body; the boss is coaxial with the connection portion; a first air hole coaxial with the boss is further defined in a central portion of the boss.

In the present application, an electronic cigarette is further provided, which comprises an atomization assembly, the atomization assembly includes an outer sleeve and a suction nozzle cover inserted at one end of the outer sleeve; wherein a sealing component, which is fixedly connected to the suction nozzle cover, is protruded radially from a circumferential outer side wall of the suction nozzle cover, and the outer side wall is in contact with the outer sleeve, and a hardness of the sealing component is smaller than that of the suction nozzle cover.

In the electronic cigarette of the present application, wherein the outer side wall of the suction nozzle cover is attached to an inner side wall of the outer sleeve; the sealing component extends from the outer side wall of the suction nozzle cover in a direction away from a central axis of the outer sleeve, and is further abutted against the inner side wall of the outer sleeve.

In the electronic cigarette of the present application, wherein the sealing component is coaxially sleeved on the circumferential outer side wall of the suction nozzle cover, and the sealing component and the inner side wall of the outer sleeve form interference fit.

In the electronic cigarette of the present application, wherein at least one groove adapted to the sealing component is defined on the circumferential outer side wall of the suction nozzle cover;
the sealing component is coaxially sleeved in the groove, and the sealing component and the inner wall of the outer sleeve form interference fit.

In the electronic cigarette of the present application, wherein a plurality of grooves are provided; the plurality of grooves are parallel to each other, and are separately arranged on an outer wall of the connection portion; each of the grooves is coaxial with the suction nozzle cover.

In the electronic cigarette of the present application, wherein the suction nozzle cover is fixed together with or integrated with the sealing component into one piece by means of double-shot molding or bonding; a cross-section of the sealing component is in shape of an "O", a "V", or a "Y".

In the electronic cigarette of the present application, wherein a vent pipe is defined in the outer sleeve; the vent pipe is communicated with air holes defined in the suction nozzle cover to form a smoke channel.

In the electronic cigarette of the present application, wherein the electronic cigarette further includes a battery assembly; the atomization assembly and the battery assembly are integrated with each other into one piece, or detachably connected to each other.

When implementing the electronic cigarette of the present application, the following advantageous can be achieved: the suction nozzle cover is fixed together with or integrated with the sealing component into one piece by means of double-shot molding, bonding or the like. In this way, the assembly of the suction nozzle cover is facilitated, and it is possible to avoid the leakage of the tobacco tar caused by not arranging the sealing component in place in the prior art. In this way, the suction nozzle cover has a sealing function, and a gap between the engagement surfaces of the suction nozzle cover and the outer sleeve is sealed, by which the leakage channel is cut off, and thus the leakage of the tobacco tar is prevented.

### BRIEF DESCRIPTION OF THE DRAWINGS

The present application will be further described with reference to the accompanying drawings and embodiments in the following, in the accompanying drawings:
Fig. 1 is a schematic view of an atomization assembly provided in a first preferred embodiment of the present application;
Fig. 2 is a schematic view of a suction nozzle cover of the atomization assembly shown in Fig. 1;
Fig. 3 is a schematic view of the atomization assembly shown in Fig. 1, in which case a sealing component is sleeved on the suction nozzle cover;
Fig. 4 is a schematic view of an atomization assembly provided in a second preferred embodiment of the present application;
Fig. 5 is a schematic view of a suction nozzle cover of the atomization assembly shown in Fig. 4;
Fig. 6 is a schematic view of an atomization assembly provided in a third preferred embodiment of the present application;
Fig. 7 is a schematic view of a suction nozzle cover of the atomization assembly shown in Fig. 6; and
Fig. 8 is a schematic view of an atomization assembly provided in a fourth preferred embodiment of the present application.

In figures:

| | | |
|---|---|---|
| 1 outer sleeve | 11 vent pipe; | |
| 2 suction nozzle cover | 20 cover body | 21 connection portion; |
| 22 flange | 23 boss | 24 first air hole; |
| 25 second air hole | 26 groove; | |
| 3 sealing component. | | |

### DETAILED DESCRIPTION OF THE PREFERRED EMBODIMENT

In order to make the technical features, the propose and the technical effect of the present application more clearly, the specific implemental means of the present application will now be described in detail with reference to the accompanying drawings.

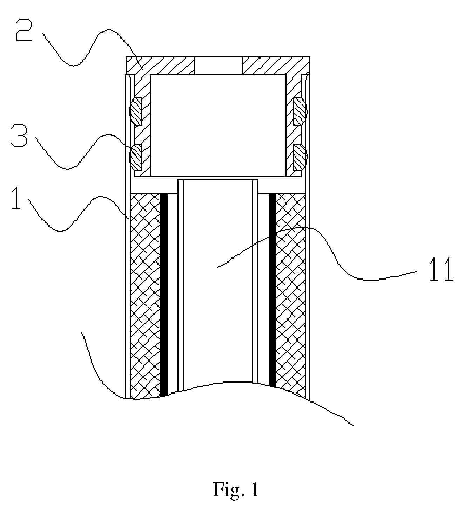

As shown in Fig. 1, in a first preferred embodiment of the present application, an atomization assembly is provided, which is configured to be assembled with a battery assembly (not shown in the figures) to form an electronic cigarette. The atomization assembly includes an outer sleeve 1, a suction nozzle cover 2 and a sealing component 3. In this case, a hardness of the sealing component is smaller than that of the suction nozzle cover 2. The outer sleeve 1 is substantially in shape of a hollow cylinder, and a vent pipe 11 or the like is defined inside the outer sleeve. The vent pipe 11 is communicated with air holes defined in the suction nozzle cover 2 to form a smoke channel.

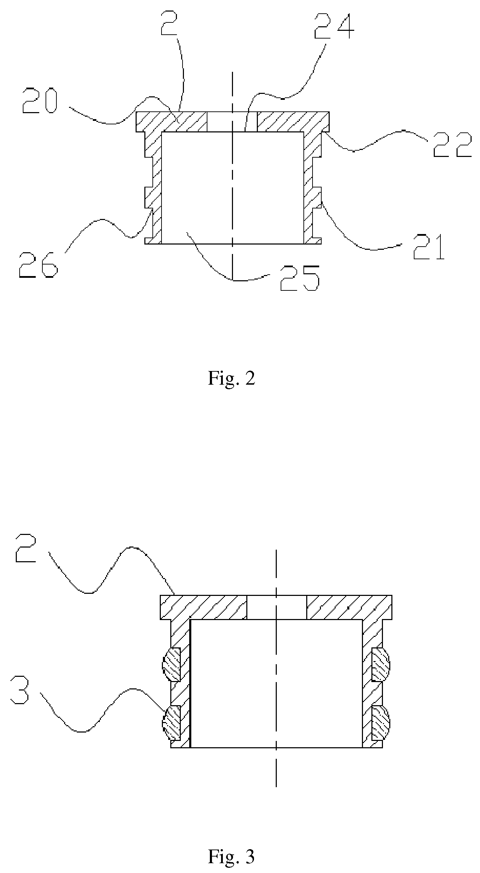

As shown in Fig. 1 and combing with Fig. 2, the suction nozzle cover 2 is substantially in shape of a hollow barrel, and the suction nozzle cover 2 is further embedded in one end of the outer sleeve 1, the end is close to the vent pipe 11. A sealing component 3, which is fixedly connected to the suction nozzle cover 2, is protruded radially from a circumferential outer side wall of the suction nozzle cover 2 in contact with the outer sleeve 1. The outer side wall of the suction nozzle cover 2 is attached to an inner side wall of the outer sleeve 1. The sealing component 3 extends from the outer side wall of the suction nozzle cover 2 in a direction away from a central axis of the outer sleeve 1, and is further abutted against the inner side wall of the outer sleeve 1.

As shown in Fig. 2, the suction nozzle cover 2 includes a cover body 20 opposite to an opening end face of the outer sleeve 1, and a connection portion 21 embedded in the outer sleeve 1. The cover body 20 is integrated with the connection portion 21.

The suction nozzle cover 2 further includes a flange 22, a first air hole 24, a second air hole 25 and a groove 26. In this case, the connection portion 21 is arranged at an edge portion of one end face of the cover body 20 that faces towards the vent pipe 11, and further extends along an axis direction of the cover body 20. A cross-section of the connection portion 21 and a cross-section of the cover body 20 are of the same shape. The connection portion 21 is substantially in shape of a hollow barrel adapted to the outer sleeve 1. The connection portion 21 is inserted into the outer sleeve 1 adapted thereto, such that the connection portion 21 is connected to the outer sleeve 1 by a plug connection. A second air hole 25 configured for the smoke to flow through is further defined in the connection portion 21.

Furthermore, a flange 22 is formed by extending from an edge of the cover body 20 in a radial direction of the cover body 20. When the connection portion 21 is inserted into the outer sleeve 1 adapted thereto, the flange 22 is abutted against a side edge of the opening end face of the outer sleeve 1, and thus the suction nozzle cover 2 is effectively prevented from sliding into the outer sleeve 1. A first air hole 24 is further defined in the cover body 20. In this case, the first air hole 24 is a hole in shape of a cylinder, and an axis thereof is aligned with or deviated from the central axis of the outer sleeve 1. In this embodiment, an axis of the first air hole 24 is preferably aligned with the central axis of the outer sleeve 1. In this case, the vent pipe 11, the second air hole 25 and the first air hole 24 are communicated with each other to form a smoke channel, in order to provide smoke for a user to suck in.

As shown in Fig. 3 combining with Fig. 2, at least one groove 26 adapted to the sealing component 3 is defined on a circumferential outer side wall of the connection portion 21, in order to reliably fix the sealing component 3. When a plurality of grooves 26 are provided, the plurality of grooves 26 are parallel to each other, and are separately arranged on an outer wall of the connection portion 21, and a central axis of each of the grooves 26 is aligned with a central axis of the connection portion 21. The sealing component 3 is coaxially sleeved in the groove 26; besides, the sealing component 3 and the inner wall of the outer sleeve 1 form interference fit. A cross-section of the sealing component 3 is in various shapes. In this case, the cross-section of the sealing component 3 may be designed in a shape adapted to the groove 26 and the outer sleeve 1, according to the structures of the groove 26 and the outer sleeve 1. For example, the cross-section of the sealing component 3 in this case may be in shape of an "O", a "V", a "Y" or the like. It can be understood that, in other embodiments, it is also possible for the connection portion 21 to have no grooves 26 defined therein, and the sealing component 3 is directly coated on a whole peripheral face of the connection portion 21.

During the practical producing process, the suction nozzle cover 2 is fixed together with or integrated with the sealing component 3 into one piece by means of double-shot molding, bonding or the like. In this way, the assembly of the suction nozzle cover 2 is simplified, and it is possible to avoid the leakage of the tobacco tar caused by not arranging the sealing component in place. When employing this structure, the suction nozzle cover 2 may be made from hard gel materials such as plastic, while the sealing component 3 may be made from soft gel materials such as silicon.

The atomization assembly provided in the first present embodiment of the present application is configured to be assembled with the battery assembly (not shown in figures) to form an electronic cigarette. It is possible for the atomization assembly to be integrated with the battery assembly, that is, the atomization assembly and the battery assembly share a same outer sleeve. However, in other embodiments, it is also possible for the atomization assembly to be detachably connected to the battery assembly. The battery assembly in this case belongs to prior art, and will not be described in detail here.

During the assembly process, the suction nozzle cover 2 sleeved with the sealing component 3 is inserted into the outer sleeve 1, and the sealing component 3 and the outer sleeve 1 form interference fit. In this way, a gap is effectively avoided from being formed between the suction nozzle 2 and the outer sleeve 1, and the tobacco tar is prevented from leaking out.

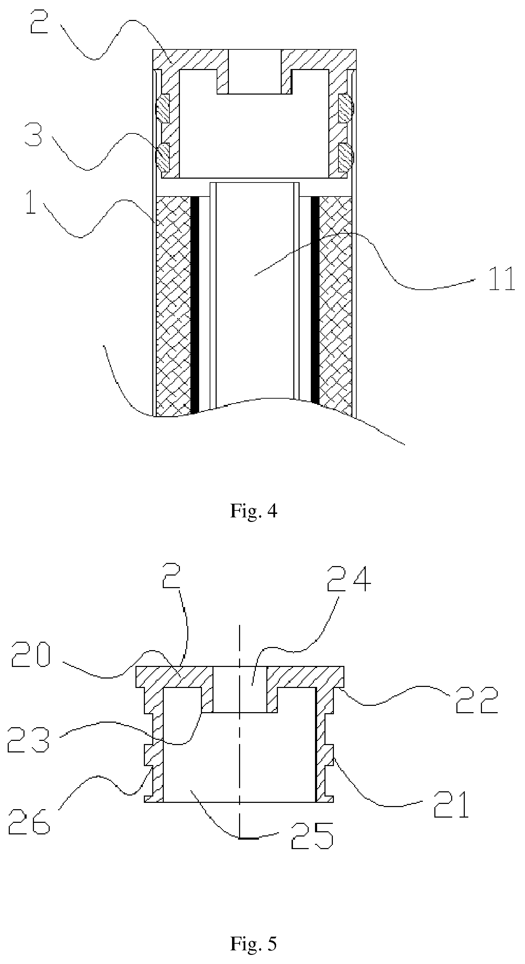

Fig. 4 shows an atomization assembly according to a second preferred embodiment of the present application, and the atomization assembly is configured to be assembled with a battery assembly to form an electronic cigarette. The differences between the atomization assembly in the second embodiment and that in the first embodiment lie in that, the structures of the cover bodies 20 are different.

As shown in Fig. 5, a boss 23 coaxial with the outer sleeve 1 is formed on a central portion on an end face of the cover body 20, the end face faces to the atomization assembly, and extends in an axis direction of the cover body 20. The boss 23 is in shape of a cylinder, and is cooperated with the second air hole 25 described above to achieve a secondary condensation and recycle of the tobacco tar. A first air hole 24 coaxial with the boss 23 is further defined in a central portion of the boss 23. The first air hole 24 is in shape of a cylinder. In this case, the vent pipe 11, the second air hole 25 and the first air hole 24 are communicated with each other to form a smoke channel, in order to provide smoke for a user to suck in.

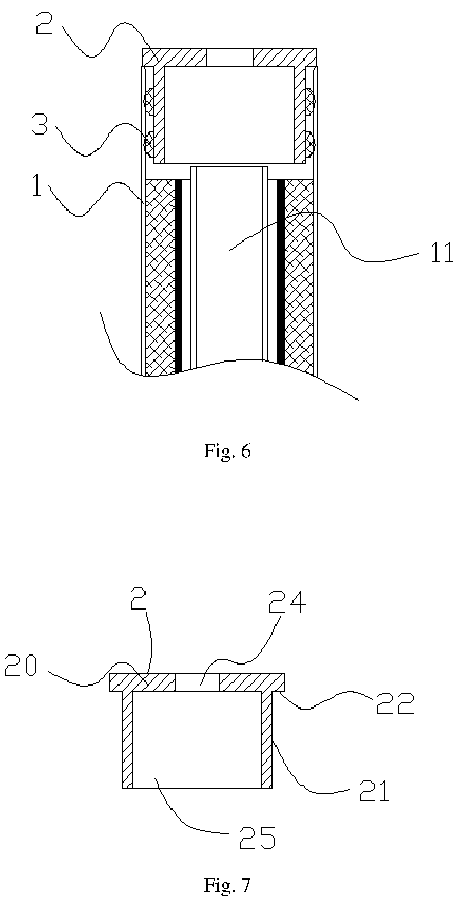

Fig. 6 shows an atomization assembly according to a third preferred embodiment of the present application, and the atomization assembly is configured to be assembled with a battery assembly to form an electronic cigarette. The differences between the atomization assembly in the third embodiment and that in the first embodiment lie in that, the structures of the connection portions 21 are different.

As shown in Fig. 6 and combing with Fig. 7, the sealing component 3 is coaxially sleeved on a circumferential outer side wall of the connection portion 21, and the sealing component 3 and an inner side wall of the outer sleeve 1 form interference fit. The connection portion 21 is fixed together with or integrated with the sealing component 3 into one piece by means of double-shot molding, bonding or the like.

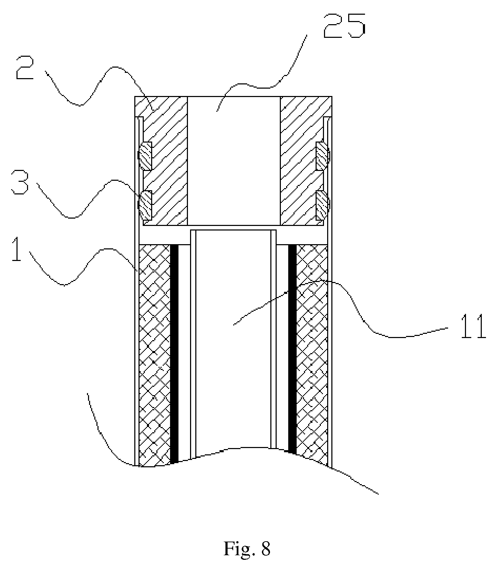

Fig. 8 shows an atomization assembly according to a fourth preferred embodiment of the present application, and the atomization assembly is configured to be assembled with a battery assembly to form an electronic cigarette. The differences between the atomization assembly in the third embodiment and that in the first embodiment lie in that, the structures of the suction nozzle covers 2 are different.

As shown in Fig. 8, the suction nozzle cover 2 is in shape of a hollow cylinder adapted to the outer sleeve 1, and a second air hole 25 is defined in the suction nozzle cover 2. The vent pipe 11 and the second air hole 25 are communicated with each other to form a smoke channel, in order to provide smoke for a user to suck in.

Although the present application is illustrated with the embodiments accompanying the drawings, however, it should be understood that, those skilled in the art may make many alternatives or equivalents, without going beyond the scope the claims intend to protect of the present application. Besides, many modifications may be made aiming at specific situation or materials, without going beyond the scope the claims intend to protect of the present application. Therefore, the present application is not limited to the specific embodiments disclosed herein, and the present application should include all the implementations fallen in the protection scope of the claims of the present application.

## Claims

1. An atomization assembly, configured to be assembled with a battery assembly to form an electronic cigarette, comprising an outer sleeve (1) and a suction nozzle cover (2) inserted at one end of the outer sleeve (1); wherein a sealing component (3), which is fixedly connected to the suction nozzle cover (2), is protruded radially from a circumferential outer side wall of the suction nozzle cover (2), and the outer side wall is in contact with the outer sleeve (1), and a hardness of the sealing component (3) is smaller than that of the suction nozzle cover (2).

2. The atomization assembly according to claim 1, wherein the outer side wall of the suction nozzle cover (2) is attached to an inner side wall of the outer sleeve (1); the sealing component (3) extends from the outer side wall of the suction nozzle cover in a direction away from a central axis of the outer sleeve (1), and is further abutted against the inner side wall of the outer sleeve (1).

3. The atomization assembly according to claim 2, wherein the sealing component (3) is coaxially sleeved on the circumferential outer side wall of the suction nozzle cover (2), and the sealing component (3) and the inner side wall of the outer sleeve (1) form interference fit.

4. The atomization assembly according to claim 2, wherein at least one groove (26) adapted to the sealing component (3) is defined on the circumferential outer side wall of the suction nozzle cover (2);
the sealing component (3) is coaxially sleeved in the groove (26), and the sealing component (3) and the inner wall of the outer sleeve (1) form interference fit.

5. The atomization assembly according to claim 4, wherein a plurality of grooves (26) are provided; the plurality of grooves (26) are parallel to each other, and are separately arranged on an outer wall of a connection portion (21); each of the grooves (26) is coaxial with the suction nozzle cover (2).

6. The atomization assembly according to claim 2, wherein the suction nozzle cover (2) is fixed together with or integrated with the sealing component (3) into one piece by means of double-shot molding or bonding; a cross-section of the sealing component (3) is in shape of an "O", a "V", or a "Y".

7. The atomization assembly according to claim 1, wherein a vent pipe (11) is defined in the outer sleeve (1); the vent pipe (11) is communicated with air holes defined in the suction nozzle cover (2) to form a smoke channel.

8. The atomization assembly according to claim 7, wherein the suction nozzle cover (2) is in shape of a hollow barrel adapted to the outer sleeve (1);the air holes are defined in the suction nozzle cover (2), and the air holes are second air holes (25).

9. The atomization assembly according to claim 7, wherein the suction nozzle cover (2) includes a cover body (20) opposite to an opening end face of the outer sleeve (1), and a connection portion (21) embedded in the outer sleeve (1).

10. The atomization assembly according to claim 9, wherein the connection portion (21) is in shape of a hollow cylinder adapted to the outer sleeve (1); a second air hole (25) is defined in the connection portion (21);
the air holes further include a first air hole (24) communicated with the second air hole (25); the first air hole (24) is defined on an end face of the cover body (20), and a central axis of the first air hole (24) is aligned with or deviated from a central axis of the outer sleeve (1);
a flange (22) is formed by extending from an edge of the cover body (20) in a radial direction of the cover body (20); the flange (22) is abutted against a side edge on an end opening face of the outer sleeve (1).

11. The atomization assembly according to claim 9, wherein a boss (23) is formed by extending from a central portion on an end face of the cover body (20) that faces to the vent pipe (11) in an axis direction of the cover body (20); the boss (23) is coaxial with the connection portion (21); a first air hole (24) coaxial with the boss (23) is further defined in a central portion of the boss (23).

12. An electronic cigarette, comprising an atomization assembly, the atomization assembly including an outer sleeve (1) and a suction nozzle cover (2) inserted at one end of the outer sleeve (1); wherein a sealing component (3), which is fixedly connected to the suction nozzle cover (2), is protruded radially from a circumferential outer side wall of the suction nozzle cover (2), and the outer side wall is in contact with the outer sleeve (1), and a hardness of the sealing component (3) is smaller than that of the suction nozzle cover (2).

13. The electronic cigarette according to claim 12, wherein the outer side wall of the suction nozzle cover (2) is attached to an inner side wall of the outer sleeve (1); the sealing component (3) extends from the outer side wall of the suction nozzle cover in a direction away from a central axis of the outer sleeve (1), and is further abutted against the inner side wall of the outer sleeve (1).

14. The electronic cigarette according to claim 13, wherein the sealing component (3) is coaxially sleeved on the circumferential outer side wall of the suction nozzle cover (2), and the sealing component (3) and the inner side wall of the outer sleeve (1) form interference fit.

15. The electronic cigarette according to claim 13, wherein at least one groove (26) adapted to the sealing component (3) is defined on the circumferential outer side wall of the suction nozzle cover (2);
the sealing component (3) is coaxially sleeved in the groove (26), and the sealing component (3) and the inner wall of the outer sleeve (1) form interference fit.

16. The electronic cigarette according to claim 15, wherein a plurality of grooves (26) are provided; the plurality of grooves (26) are parallel to each other, and are separately arranged on an outer wall of a connection portion (21); each of the grooves (26) is coaxial with the suction nozzle cover (2).

17. The electronic cigarette according to claim 13, wherein the suction nozzle cover (2) is fixed together with or integrated with the sealing component (3) into one piece by means of double-shot molding or bonding; a cross-section of the sealing component (3) is in shape of an "O", a "V", or a "Y".

18. The electronic cigarette according to claim 12, wherein a vent pipe (11) is defined in the outer sleeve (1); the vent pipe (11) is communicated with air holes defined in the suction nozzle cover (2) to form a smoke channel.

19. The electronic cigarette according to claim 12, wherein the electronic cigarette further includes a battery assembly; the atomization assembly and the battery assembly are integrated with each other into one piece, or detachably connected to each other.
